# EUROPÄISCHE PATENTANMELDUNG

(11) **EP 1 438 931 A1**
(43) Veröffentlichungstag der Anmeldung: **21.07.2004**
(21) Anmeldenummer: 04000672.8
(22) Anmeldetag: 15.01.2004
(51) Int. Cl.: A61F 2/18

(54) **Flexible Gehörknöchelchenprothese**

(30) Priorität: 17.01.2003 DE 20300723 U
(71) Anmelder: Heinz Kurz GmbH Medizintechnik, 72144 Dusslingen (DE)
(72) Erfinder: Steinhardt, Uwe, 72145 Hirrlingen (DE); Hüttenbrink, Karl-Bernd, Prof. Dr. med, 01326 Dresden (DE)
(74) Vertreter: Möbus, Daniela, Dr.-Ing.

(57) **Zusammenfassung**

Eine Gehörknöchelchenprothese mit einer Kopfplatte, die am Trommelfell anliegt und einem Basisteil, das den Steigbügel aufnimmt oder im ovalen Fenster auf der Steigbügelfußplatte aufliegt, wobei die Kopfplatte (10) und das Basisteil (12) beweglich mittels eines flexiblen Drahts (14) verbunden sind.

## Beschreibung

Die Erfindung betrifft eine flexible Gehörknöchelchenprothese.

Gehörknöchelchenprothesen werden verwendet, um bei ganz oder teilweise fehlenden oder beschädigten Gehörknöchelchen des menschlichen Mittelohrs den Schall vom Trommelfell, das das Außenohr vom Mittelohr trennt, zum ovalen Fenster, das das Mittelohr vom Innenohr trennt, zu übertragen. Es wird dabei zwischen einer Partialprothese und einer Totalprothese unterschieden. Bei einer Partialprothese wird der Steigbügel der Gehörknöchelchen, der am ovalen Fenster anliegt, mitverwendet, sodass die Prothese an einem Ende ein Basisteil aufweist, das den Steigbügel aufnimmt, und die am anderen Ende eine Kopfplatte besitzt, mit der sie am Trommelfell anliegt. Bei einer Totalprothese ersetzt diese alle Gehörknöchelchen. Das Basisteil der Prothese liegt nun direkt am ovalen Fenster bzw. auf der Steigbügelfußplatte an und die Kopfplatte der Prothese am Trommelfell.

Da die anatomischen Gegebenheiten des Ohrs, wie beispielsweise die Lage und Form des Steigbügels, des Trommelfells und des ovalen Fensters, variieren, und da es sein kann, dass auch nach einer Operation, bei der die Gehörknöchelchenprothese eingesetzt wurde, noch Lageveränderungen durch den Heilungsprozess durch Zugkräfte in den Gewebestrukturen auftreten können, ist es vorteilhaft, wenn die Gehörknöchelchenprothese eine gewisse Flexibilität aufweist.

Der Erfindung liegt daher die Aufgabe zugrunde, eine Gehörknöchelchenprothese so auszubilden, dass sie in zuverlässiger, einfacher und verschleißfreier Weise eine gewisse Flexibilität aufweist.

Die gestellte Aufgabe wird erfindungsgemäß durch die Gehörknöchelchenprothese, die die im Hauptanspruch aufgeführten Merkmale aufweist, gelöst. Die Unteransprüche geben bevorzugte Weiterbildungen an.

Bei der erfindungsgemäßen Gehörknöchelchenprothese sind die Kopfplatte, die am Trommelfell anliegt, und das Basisteil, das bei einer Partialprothese entweder den Steigbügel aufnimmt oder bei einer Totalprothese im ovalen Fenster auf der Steigbügelfußplatte aufliegt, durch einen flexiblen Draht beweglich miteinander verbunden.

In einer bevorzugten Ausführungsform besteht dieser Draht aus Nitinol oder einem federnden Stahl.

Je nach erwünschtem Grad der Flexibilität und Stabilität kann der Draht unterschiedliche Durchmesser aufweisen. Durchmesser, die im Bereich von 0,05 mm bis 0,4 mm liegen, haben sich als besonders geeignet herausgestellt.

Je nach der Ausgestaltung der Kopfplatte und des Basisteils kann der sie verbindende Draht an verschiedenen Stellen der Kopfplatte und des Basisteils befestigt werden. In den meisten Fällen wird wohl eine Befestigung in der Mitte am vorteilhaftesten sein.

Bei einer Partialprothese muss das Basisteil den Steigbügel aufnehmen. Es kann dazu in Form einer Glocke ausgebildet sein und es kann auch einen federnden Clip aufweisen, mit dem es auf sichere Weise am Steigbügel befestigt werden kann.

Alle Materialien der Gehörknöchelchenprothese müssen, um Abstoßungsreaktionen zu vermeiden, aus einem biokompatiblen Material hergestellt sein. Vorzugsweise wird als Material Titan, eine Titanlegierung, Nitinol oder Stahl verwendet.

Ein Ausführungsbeispiel einer erfindungsgemäß ausgebildeten Gehörknöchelchenprothese wird nachfolgend anhand der beiliegenden Zeichnung erläutert. In den Zeichnungen sind gleiche Elemente in allen Zeichnungsfiguren mit den gleichen Bezugszahlen gekennzeichnet.

Es zeigen:
- Fig. 1: eine perspektivische Darstellung eines Ausführungsbeispiels einer Gehörknöchelchenpartialprothese; und
- Fig. 2: eine perspektivische Darstellung eines Ausführungsbeispiels einer Gehörknöchelchentotalprothese.

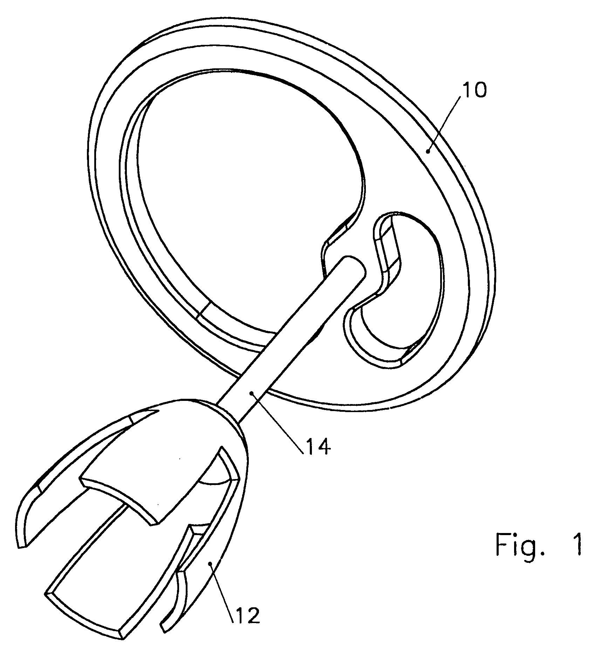

Fig. 1 zeigt in perspektivischer Darstellung eine Ausführungsform einer Gehörknöchelchenpartialprothese. Sie weist an ihrem einen Ende, wo sie am Trommelfell anliegt, eine Kopfplatte 10 auf. Die Kopfplatte 10 kann unterschiedliche Formen aufweisen und muss auch nicht vollflächig ausgeführt sein.

An ihrem anderen Ende weist die Gehörknöchelchenpartialprothese ein Basisteil 12 auf. Das Basisteil 12 muss bei einer Partialprothese den Steigbügel aufnehmen. Es ist daher beim hier dargestellten Ausführungsbeispiel in Form einer Glocke mit durchbrochenen Wänden ausgebildet.

Die Kopfplatte 10 und das Basisteil 12 sind erfindungsgemäß durch einen flexiblen Draht 14 verbunden. Der flexible Draht 14 ist im hier dargestellten Ausführungsbeispiel außermittig an der Kopfplatte 10 befestigt.

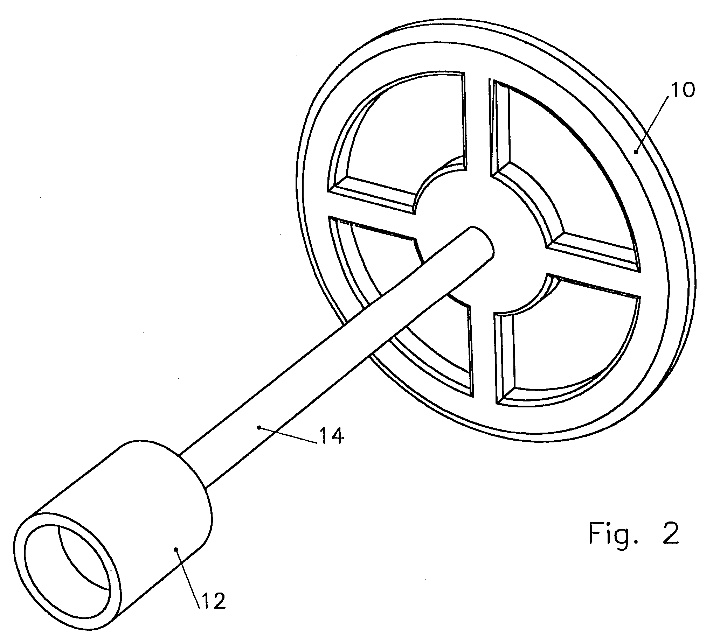

Fig. 2 zeigt in perspektivischer Darstellung eine Ausführungsform einer Gehörknöchelchentotalprothese. Sie weist an ihrem einen Ende, wo sie am Trommelfell anliegt, wiederum eine Kopfplatte 10 auf. Die Kopfplatte 10 ist hier in Form eines mit Achsen versehenen Kreises ausgebildet.

Bei der Gehörknöchelchentotalprothese liegt das Basisteil 12 direkt oder über eine hier nicht dargestellte Steigbügelfußplatte am ovalen Fenster an und es ist daher hier in Form eines Stempels ausgebildet.

Kopfplatte 10 und Basisteil 12 sind auch hier wieder erfindungsgemäß durch einen flexiblen Draht 14 verbunden. Der flexible Draht 14 ist im hier dargestellten Ausführungsbeispiel mittig an der Kopfplatte 10 und am Basisteil 12 befestigt.

### Bezugszeichenliste:

- 10: Kopfplatte
- 12: Basisteil
- 14: flexibler Draht

## Patentansprüche

1. Gehörknöchelchenprothese mit einer Kopfplatte, die am Trommelfell anliegt und einem Basisteil, das den Steigbügel aufnimmt oder im ovalen Fenster auf der Steigbügelfußplatte aufliegt, **dadurch gekennzeichnet, dass** die Kopfplatte (10) und das Basisteil (12) beweglich mittels eines flexiblen Drahts (14) verbunden sind.

2. Gehörknöchelchenprothese nach Anspruch 1, **dadurch gekennzeichnet, dass** der Draht (14) aus Nitinol oder federndem Stahl besteht.

3. Gehörknöchelchenprothese nach Anspruch 1 oder 2, **dadurch gekennzeichnet, dass** der Draht (14) einen Durchmesser von 0,05 bis 0,4 mm aufweist.

4. Gehörknöchelchenprothese nach einem der vorherigen Ansprüche, **dadurch gekennzeichnet, dass** der Draht (14) in der Mitte der Kopfplatte (10) und des Basisteils (12) befestigt ist.

5. Gehörknöchelchenprothese nach einem der vorherigen Ansprüche, **dadurch gekennzeichnet, dass** das Basisteil (12), wenn es zur Aufnahme des Steigbügels dient, in Form einer Glocke ausgebildet ist.

6. Gehörknöchelchenprothese nach einem der vorherigen Ansprüche, **dadurch gekennzeichnet, dass** das Basisteil (12), wenn es zur Aufnahme des Steigbügels dient, einen federnden Clip umfasst.

7. Gehörknöchelchenprothese nach einem der vorherigen Ansprüche, **dadurch gekennzeichnet, dass** die Kopfplatte (10) und das Basisteil (12) aus einem biokompatiblen Material, vorzugsweise aus Titan, einer Titanlegierung, Nitinol oder Stahl bestehen.
